# EUROPEAN PATENT SPECIFICATION

(11) **EP 1 618 835 B1**
(45) Date of publication and mention of the grant of the patent: **28.08.2013**
(21) Application number: 04728674.5
(22) Date of filing: 21.04.2004
(51) Int. Cl.: A61B 1/06

(54) **CAPSULE ENDOSCOPE AND CAPSULE ENDOSCOPE SYSTEM**
KAPSELENDOSKOP UND KAPSELENDOSKOPSYSTEM
ENDOSCOPE A CAPSULE

(30) Priority: 25.04.2003 JP 2003122807
(43) Date of publication of application: 25.01.2006
(73) Proprietor: OLYMPUS CORPORATION, Shibuya-ku Tokyo 151-0072 (JP)
(72) Inventor: KIMOTO, Seiichiro, Tokyo 1920045 (JP); FUJIMORI, Noriyuki, Nagano 3920131 (JP); SUZUSHIMA, Hiroshi, Kamiina-gun, Nagano, 3994601 (JP); SHIGEMORI, Toshiaki, Kanagawa 2140034 (JP); NAKAMURA, Tsutomu, Nagano 3994511 (JP); NAGASE, Ayako, Tokyo 1920045 (JP); MINAI, Tetsuo, Tokyo 1920023 (JP); SHIMIZU, Hatsuo, Tokyo 1920024 (JP); HONDA, Takemitsu, Tokyo 1910062 (JP); SASAGAWA, Katsuyoshi, Tokyo 1910041 (JP); SUZUKI, Katsuya, Kanagawa 2291103 (JP); HASHIMOTO, Masayuki, Nara 6310806 (JP); ORIHARA, Tatsuya, Tokyo 1920023 (JP); NAKATSUCHI, Kazutaka, Tokyo 1910062 (JP)
(74) Representative: von Hellfeld, Axel
(86) International application number: PCT/JP2004/005710
(87) International publication number: WO 2004/096029

(56) References cited:
- WO-A1-01/35813
- GB-A- 2 011 111
- JP-A- 4 102 432
- JP-A- 5 060 985
- JP-A- 11 076 151
- JP-A- 2003 093 367
- JP-U- 2 104 315
- US-A1- 2003 023 150
- US-B1- 6 251 068

## Description

### TECHNICAL FIELD

The present invention relates to endoscopes for medical use, and more particularly, relates to a swallowable capsule-shaped endoscope (hereinafter, "capsule endoscope'') and a capsule endoscope system.

### BACKGROUND ART

A capsule endoscope which contains an illuminating unit in a capsule casing having a size that a patient can swallow, where the illuminating unit uses, for example, an image capturing unit includes a solid-state image sensor, or an LED, has been known.

Such a capsule endoscope can capture images of the inside of an abdominal cavity such as a stomach and intestines. when passing through the abdominal cavity after a patient swallows it.

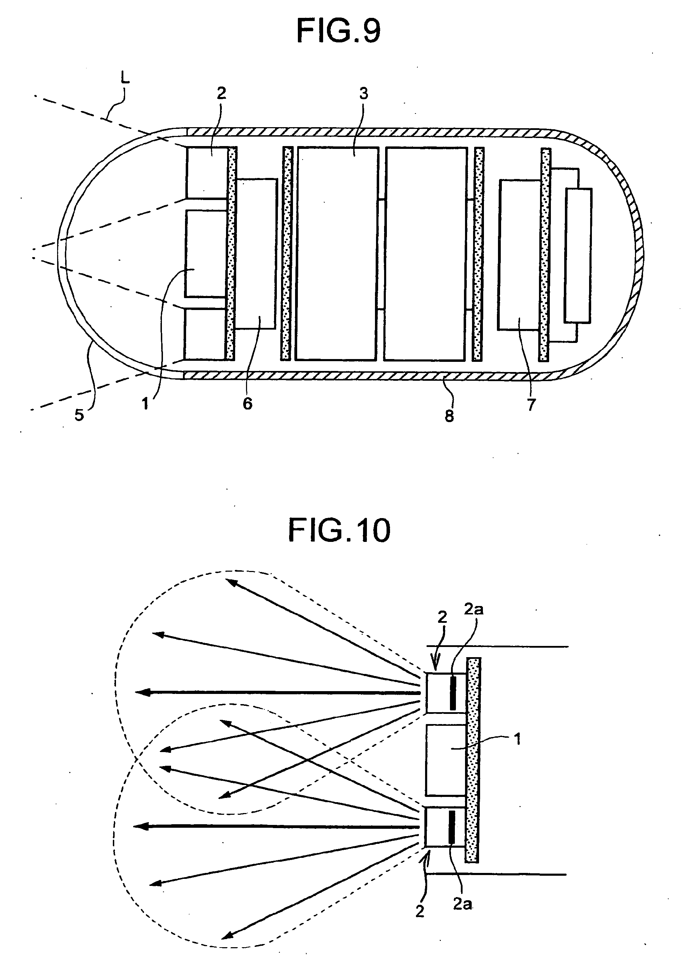

An example of the capsule endoscope is described below with reference to Fig. 9.

As shown in Fig. 9, the conventional capsule endoscope includes an image capturing unit 1 that captures images of the inside of an abdominal cavity, an illuminating unit 2 that illuminates the inside of the abdominal cavity, a power-supply unit 3 that supplies power to these units, a front cover 5 that covers the image capturing unit 1 and the illuminating unit 2 and guides the light L from the illuminating unit 2 to outside, and a capsule casing 8 that is assembled to retain watertight sealing between the capsule casing 8 and front cover 5 and that houses at least the image capturing unit 1, the illuminating unit 2, the power-supply unit 3, and processing circuits 6 and 7.

The illuminating unit 2 can illuminate the inside of the abdominal cavity while the image capturing unit 1 captures images of the inside of the abdominal cavity. The illuminating unit 2 and the image capturing unit 1 are generally disposed near each other in facing forward. Provided at the top of the capsule casing 8 is the front cover 5 having a semispherical shape, serving as an illumination guiding window and an abdominal cavity capturing window, and being partly or fully transparent, to guide the illumination light to the inside of the abdominal cavity and to allow the image capturing unit to capture images of the inside of the abdominal cavity.

The semispherical shape of the front cover 5 allows a patient to easily swallow the capsule endoscope and makes it difficult for body fluids and the like to attach on the front cover 5 (see Japanese Patent Application Laid-open Publication No. 2001-95756).

However, the optical axis (outgoing light represented by a bold line in the figure) of the illuminating unit 2 disclosed in Japanese Patent Application Laid-open Publication No. 2001-95756 is parallel to the axis of the capsule endoscope, and the radiant light from the radiating unit is equally radiated like an inverted cone. Therefore, if a plurality of illuminating units 2 are coaxially arranged as shown in Fig. 10, the central area of the illuminated range are illuminated with a total of illumination light from the plural illuminating units, so that the central area is different from the other area in brightness.

As a result, there is a problem that the brightness is unbalanced and thus the image capturing unit cannot capture good diagnosis images.

Document US 2003/0023150 A1 concerns a capsule-type medical device comprising an image pick-up device around which LEDs are arranged.

Document WO 00/76391 A1 concerns an optical system for illuminating and viewing a target. In one embodiment, the system comprises a plurality of illumination elements positioned on a focal curve to enable a uniform spatial illumination.

An object of the present invention is to provide a capsule endoscope that can uniformly illuminate a whole surface to be illuminated with illumination light and capture good diagnosis images in light of the above problem.

### DISCLOSURE OF INVENTION

To solve the above-mentioned problems and achieve the above-mentioned objects a capsule endoscope having the features of claim 1 is provided.

A capsule endoscope comprises an observation unit having a predetermined observation range to observe an inside of a body of a patient; and a plurality of illuminating units arranged around the observation unit, each of the illuminating units emitting illuminating light for illuminating the observation range of the observation unit. Each of the illuminating units have a light distribution characteristics that deviates from the center of the observation range of the observation unit. The illuminating light emitted from the illuminating units overlaps at a central portion of the observation range of the observation unit so that an amount of the light at the central portion of the observation range is substantially the same as an amount of light at a portion around the central portion of the observation range.

The observation unit may include an optical element having a predetermined optical axis for deciding the observation range and the center of the observation range, and forming an image of the inside of the body of the patient; and an image capturing element capturing the image formed by the optical element.

Each of the illuminating units may be a light emitting element including a light emitting body emitting illuminating light for illuminating the observation range of the observation unit.

The light emitting body in each of the illuminating units my be arranged to tilt so that an end of the light emitting body close to the center of the observation range is disposed upward, to overlap the illuminating light emitted from each light emitting body at the central portion of the observation range.

Each of the illuminating units may be arranged to tilt so that an end of the illuminating unit close to the center of the observation range is disposed upward, to overlap the illuminating light emitted from each of the illuminating units at the central portion of the observation range.

The invention further comprises a reflecting member adjacent to each of the illuminating units. Each of the illuminating units also overlaps illuminating light reflected by the reflecting member in addition to the illuminating light emitted from each of the illuminating units, at the central portion of the observation range.

A capsule endoscope comprises an image capturing unit having a predetermined optical axis and a vision range, and capturing an image of an inside of a body of a patient; and a plurality of illuminating units arranged around the image capturing unit. Each of the illuminating units emits illuminating light for illuminating the vision range of the image capturing unit. Each of the illuminating units has a light distribution characteristics that deviates from the optical axis of the image capturing unit. The illuminating light emitted from the illuminating units overlaps at a central portion of the observation range including an extension of the optical axis of the image capturing unit so that an amount of the light at the central portion of the observation range is substantially the same as an amount of light at a portion around the central portion of the observation range, and the central portion.

The endoscope may further comprise a communication unit transmitting image capturing information acquired by the image capturing unit to outside.

### BRIEF DESCRIPTION OF DRAWINGS

Fig. 1 is a schematic diagram of the internal structure of a capsule endoscope;
Fig. 2 is a schematic diagram of a capsule endoscope system;
Fig. 3 is a front view of the capsule endoscope;
Fig. 4 is a schematic diagram for explaining emission of illumination light from an illuminating unit;
Fig. 5 is a schematic diagram showing the intensity of the illumination light emitted from the illuminating unit, viewed from the front of the capsule endoscope;
Fig. 6 is a schematic diagram for explaining emission of illumination light from an illuminating unit;
Fig. 7 is a schematic diagram for explaining emission of illumination light from an illuminating unit according to the invention;
Fig. 8 is an enlarged view for explaining emission of illumination light from an illuminating unit according to the invention;
Fig. 9 is a schematic diagram of a conventional capsule endoscope; and
Fig. 10 is a schematic diagram for explaining guide for and reflection of illumination light from an illuminating unit of the conventional capsule endoscope.

### BEST MODE(S) FOR CARRYING OUT THE INVENTION

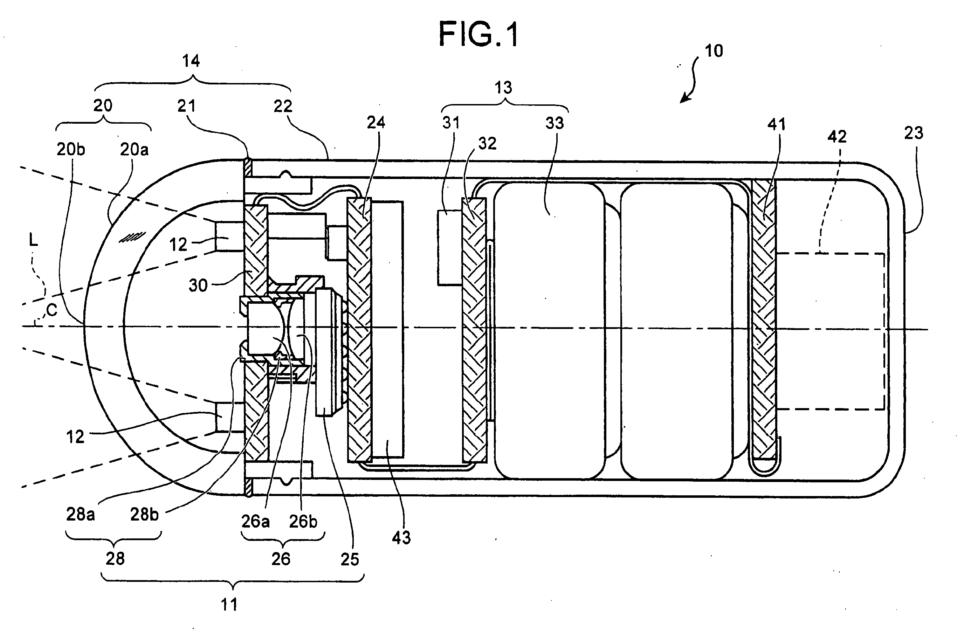

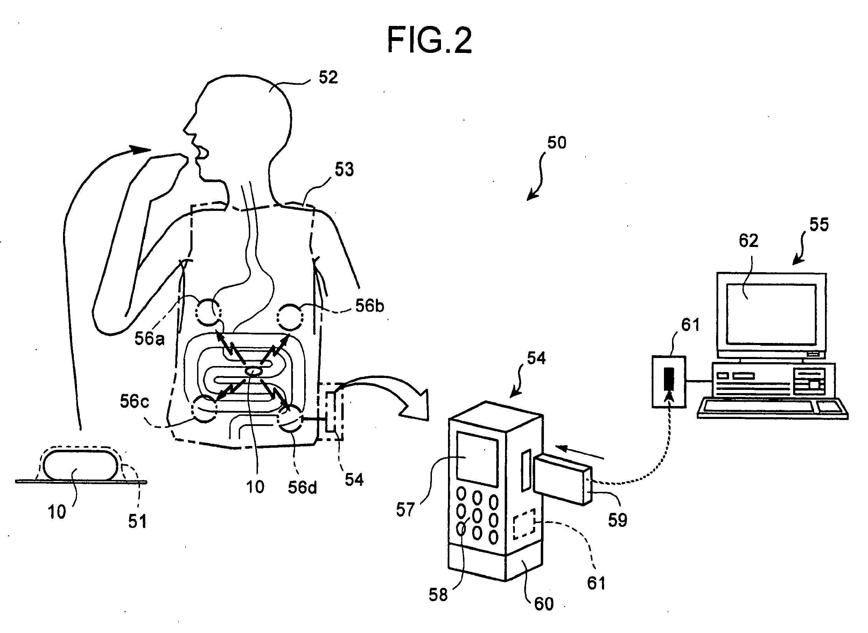

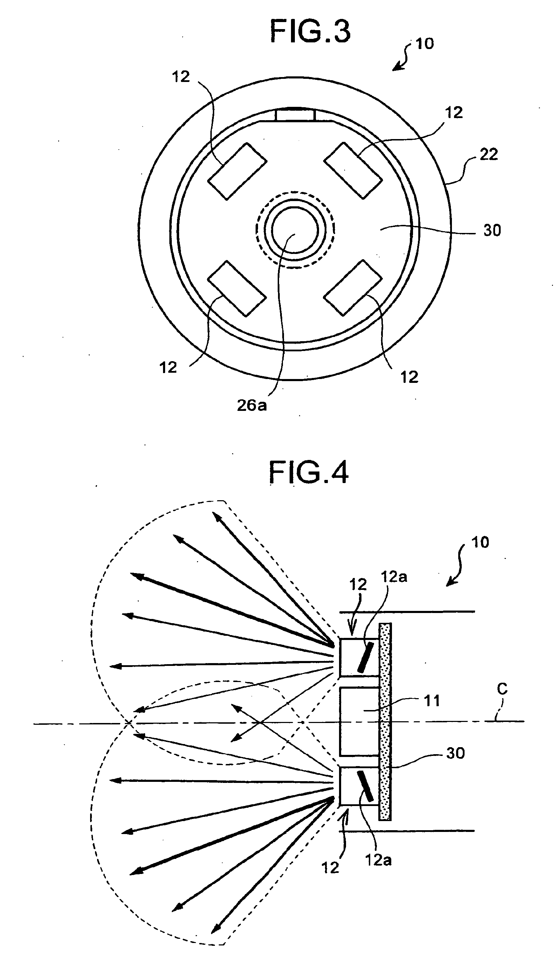

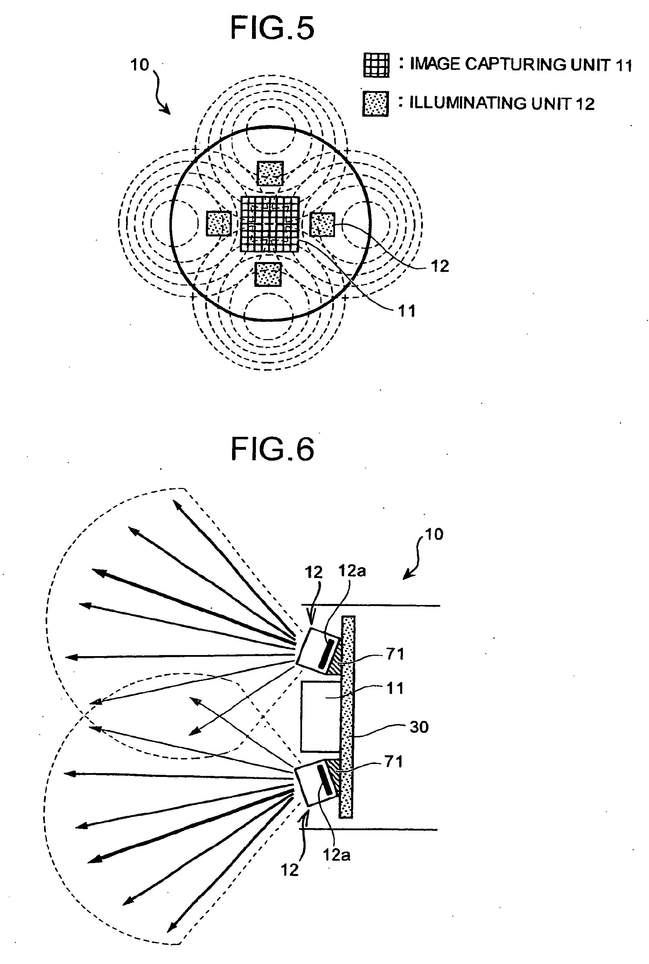

The present invention will be explained in detail below through embodiments of the invention but is not limited to those embodiments. Fig. 1 is a schematic diagram of the internal structure of a capsule endoscope; Fig. 2 is a schematic diagram of a capsule endoscope system; Fig. 3 is a front view of the capsule endoscope; Fig. 4 is a schematic diagram for explaining emission of illumination light from an illuminating unit according to the embodiment; and Fig. 5 is a schematic diagram showing the intensity of the illumination light emitted from the illuminating unit, viewed from the front of the capsule endoscope.

Fig. 1 is a schematic diagram of the internal structure of a capsule endoscope; and Fig. 2 is a schematic diagram of a capsule endoscope system.

An overall internal structure of the capsule endoscope 10 is first described with reference to Fig. 1. The capsule endoscope 10 includes an image capturing unit 11 that is an observation unit for capturing images of the inside of an abdominal cavity, an illuminating unit 12 that illuminates the inside of the abdominal cavity, a power-supply unit 13 that supplies an electric power to both units, and a watertight capsule casing 14 that houses the image capturing unit 11, the illuminating unit 12, and the power-supply unit 13 at least.

The capsule casing 14 has a front cover 20 that covers the image capturing unit 11 and the illuminating unit 12, and a capsule trunk 22 that is provided to retain watertight sealing between the capsule trunk 22 and the front cover 20 by a watertight sealer 21 and that houses the image capturing unit 11 and the like. A rear cover 23, which is separable, if required, may be provided to the capsule trunk 22. It is assumed that the rear cover 23 is provided integrally with the capsule trunk and is flat, however, its shape is not limited to but the rear cover may be dome-shaped, for example.

The front cover 20 may include an illuminating window 20a for allowing the illumination light L from the illuminating unit 12 to pass through and a capturing window 20b for capturing images in an illuminated range, each of the windows being distinct, separable. The whole of the front cover 20 is transparent and areas of the window 20a and the window 20b overlap partly.

The image capturing unit 11 is installed on an image capturing substrate 24, and includes a solid-state image sensor 25 which is, for example, a charged couple device (CCD), and which captures images in the range illuminated by the light L; and an image forming lens 26, for forming an image of an object on the solid-state image sensor 25, which includes a fixed lens 26a and a movable lens 26b. A sharp image is formed by a focusing unit 28 which includes a fixing frame 28a to firmly hold the fixed lens 26a and a movable frame 28b to movably hold the movable lens 26b.

The image capturing unit 11 is not limited to the CCD, but may be another image capturing unit such as a complementary metal-oxide semiconductor (CMOS).

The illuminating unit 12 is provided on an illuminating substrate 30 and is, for example, a light emitting diode (LED). A plurality of illuminating units 12 (four illuminating units in this embodiment) are disposed around the image forming lens 26, which constitutes the image capturing unit 11.

The power-supply unit 13 is installed on a power-supply substrate 32 that has an internal switch 31, and uses, for example, a button battery cell as a power supply 33. It is assumed here that the battery cell is a silver-oxide cell; however, the battery cell may be a rechargeable cell, a dynamo cell, or the like.

The internal switch 31 is a switch that can be made ON/OFF by repulsion of magnets.

A wireless unit 42, which includes an antenna etc., for performing wireless communication with outside is installed on a wireless substrate 41, and performs communication with the outside if required.

A signal-processing and control unit 43 that processes and controls the various units is installed on the image capturing substrate 24, and executes various processes in the capsule endoscope 10.

The signal-processing and control unit 43 includes an image-signal processing function such as image-data generation with correlated double sampling (CDS); a transmission-signal generating function to perform, for example, mixing of an image signal and a synchronization signal (in a case of analog transmission) and addition of an error correction code (in a case of digital transmission); a modulation function to convert in cooperation with a modulator, to a phase-shift keying (PSK) modulation, a minimum-shift keying (MSK) modulation, a Gaussian minimum-shift keying (GMSK) modulation, a quadrature minimum-shift keying (QMSK) modulation, and an amplitude-shift keying (ASK) modulation format, for example; a power-supply control function to control the power supply according to ON-OFF operation of a switch; a timing-generator (TG) function to control a driver circuit like an LED driver circuit and to control the number of images to be captured; and a storage function to store various data like parameters of line and frame etc., and performs various signal processing and controls.

The signal processing may include image-data correction (white balance (WB) correction, y correction, color processing, automatic gain control (AGC) etc.), analog-digital conversion (ADC), automatic exposure control function (AE), and the like.

A capsule endoscope system is explained below with reference to Fig. 2. Fig. 2 is a schematic diagram of the capsule endoscope system. The capsule endoscope system 50 shown in Fig. 2 is used for checking a patient with the capsule endoscope 10.

The capsule endoscope system 50 according to this embodiment includes, for example, the capsule endoscope 10, its package 51, a jacket 53 to be worn by a patient 52, a receiver 54 that can be detachably attached to the jacket 53, and a work station 55 that processes information received in the receiver 54.

Antennas 56a, 56b, 56c, and 56d which receive electromagnetic waves of image signals transmitted from the wireless unit 42 of the capsule endoscope 10 are installed in the jacket 53 and are provided to enable wireless communication or wired communication by a cable with the receiver 54. The number of antennas installed in the jacket 53 is not limited to four and would be two or more so that the electromagnetic waves from the location of the capsule endoscope 10, which changes by its movement, can be received properly.

The receiver 54 includes a display 57 that displays information necessary for observation (examination) and an input unit 58 to input information necessary for observation (examination). A CF (compact flash (registered trademark)) memory 59 that stores the received image data, is detachably mounted on the receiver 54. The receiver 54 is also provided with a power-supply unit 60 that can supply power even while carrying, and a signal processing and control section 61 that performs processing required for observation (examination). A dry battery cell, a lithium-ion secondary battery cell, nickel-hydrogen battery cell etc. are examples of the power-supply unit 60 and it may be a rechargeable battery cell as well.

The work station 55 has processing functions to perform diagnosis based on images of internal organs in a body of a patient which a doctor or a nurse has captured by the capsule endoscope 10. This work station 55 is provided with a CF memory reader/writer 61. It is not shown in the diagram but the receiver 54 and the CF memory reader/writer 61 have interfaces that can be connected to enable communication, and read and write the CF memory 59.

Moreover, the work station 55 has a communication function for connecting to a network, and via this network, a medical examination result of the patient is stored in a database. Further, the work station 55 has a display 62 and receives the captured image data of the inside of the patient's body from the receiver 54, and displays images of internal organs etc. on the display 62.

As shown in FIG. 2, before the examination is carried out, the capsule endoscope 10 is taken out from the package 51, and the patient 52 swallows the capsule endoscope 10. The capsule endoscope 10 passes through esophagus of the patient, advances to an abdominal cavity through peristalsis of an alimentary canal cavity, and captures images of the inside of the abdominal cavity one after another.

The result of capturing, when needed or at any time, is output as the electromagnetic waves of the captured images via the wireless unit 42, and is received by the antennas 56a to 56d of the jacket 53. The antenna that receives signals with a high electromagnetic power receiver 54 transmits the signals to the receiver 54 which is located outside the body of the patient.

The receiver 54 stores the captured image data, which is sequentially received, in the CF memory 59. The operation of the receiver 54 is not synchronized with the start of image capturing of the capsule endoscope 10, but, the start and the end of receiving are controlled by an operation of the input unit 58. The captured image data may be still-image data captured at a plurality of frames per second for displaying them as moving images or may be normal video-image data.

When the observation (examination) of the patient 52 by the capsule endoscope 10 is completed, the CF memory 59 is inserted into the CF memory reader/writer 61. The captured data in the CF memory 59 is then transferred to the work station 55. In the work station 55, the transmitted data is associated with and stored for each patient.

Thus, the captured image data of the inside of the abdominal cavity, which is captured by the capsule endoscope 10 and stored by the receiver 54, is displayed by the display 62 of the work station 55. This enables to acquire images of internal organs (e.g., a small intestine) which are not accessible by an ultrasonic probe, endoscope etc.

Fig. 4 shows the structure of the illuminating unit.

Each illuminating unit 12 includes a light emitting body 12a arranged to tilt outward from a central axis C of the capsule endoscope 20 so that the center of the optical axis of the light L emitted from each light emitting body 12a is inclined to outside. The central axis C is the center of an observation region, and also the central axis of the capsule trunk 22 which is almost cylindrical and constitutes the capsule casing 14 shown in Fig. 1. It is assumed here that the optical axis of the image forming lens 26 and the front cover 20 of the image capturing unit 11 coincides with the central axis C; however, their arrangement is not limited to. If the optical axis is different from the central axis C, each light emitting body 12a may be arranged to tilt outward from the optical axis so that the optical axis functions as the center of the observation region.

As a result, the brightness of illuminating light that illuminates the center of the observation region where the light from the illuminating units 12 gathered is substantially the same as the brightness of illuminating light at the portion other than the center of the observation region.

In other words, as shown in Fig. 5, the illuminating units 12 arranged around the image capturing unit 11, if brightness of the light L from the illuminating unit 12 is indicated by isointensity lines (the smaller the distance between the lines, the larger the amount of light), the intensity of the light is adjusted such that central light towards an outer side of the capsule endoscope rather than central side of the illuminating unit 12 is high, and the light that illuminates the center of the illuminating range (observation central portion) among the light emitted from each light emitting body 12a constituting the illuminating unit 12, is allowed to be incident in a different direction (hereinafter, "anisotropy factor of light" in the present invention) so as to be less in brightness than incident light that illuminates a portion of the illuminating range excluding the observation central portion.

As a result, a brightness of the observation central portion of the illuminating range illuminated by the plurality of light emitting bodies 12a and the brightness of the portion excluding the observation central portion of the illuminating range illuminated by a single light emitting body becomes the same or substantially the same.

Since the observation central portion of the illuminating range has a predetermined brightness due to the gathered illuminating light from the plural light emitting bodies 12a, 12a, 12a, and 12a (four in this embodiment) and an illuminating range other than the observation central portion has substantially the same brightness or the same brightness as the predetermined brightness of the observation central portion with a single light emitting body 12a, the overall illuminating range can be made to have a uniform brightness, thereby having balanced brightness. As a result, it is possible to provide the capsule endoscope that enables to capture a good diagnosis image with the image capturing unit.

Due to this, the amount of the illuminating light in an illuminated plane that is identical to a plane perpendicular to the central axis of the capsule endoscope is substantially the same.

There is example drawback in the conventional capsule endoscope that a situation where the desired brightness can be acquired in the observation central portion but not in a surrounding portion other than the observation central portion arises.

In this situation, if the power supply from the power-supply unit 13 is increased, then a portion other than the observation central portion can have the desired brightness but a portion already having the desired brightness also becomes brighter. As a result, this leads to wasteful power consumption.

In this regard, the observation central portion of the illuminating range has the desired brightness with the plural light emitting bodies and an illuminating range other than the observation central portion has substantially the same brightness or the same brightness as the desired brightness of the observation central portion with a single light emitting body. Accordingly, it is possible to prevent a situation where though the observation central portion has the desired brightness, the other portion does not have the desired brightness.

In other words, in a case where the portion other than the central portion does not have the desired brightness, the central portion does not have the desired brightness similarly. In such a case, when the power supply from the power-supply unit 13 is increased, the overall portion becomes bright. Therefore, the power supply can be used effectively and it is possible to prevent unnecessary power consumption as in the conventional case.

Fig. 6 is a schematic diagram of an illuminating unit of a capsule endoscope.

In the first example shown in Fig. 4 the light emitting bodies 12a constituting the illuminating unit 12 are tilted. In the second example, as shown in Fig. 6, the illuminating units 12 themselves are tilted by using a tilting member 71 so that the illuminating units 12 are inclined from front side towards back side in an outward direction from the central axis of the capsule endoscope.

A light emitting surface of the light emitting body 12a and a light emitting surface of the illuminating unit 12 are parallel. Therefore, there is no change incorporated in the structure of the illuminating unit 12. The existing illuminating units may be used and tilting is provided by the tilting member 71. By tilting, the observation central portion of the illuminating range is allowed to acquire a predetermined brightness due to the light gathered from the plurality of light emitting bodies and the illuminating range other than the observation central portion is allowed to acquire substantially the same or the same brightness as the predetermined brightness of the central portion by using a single light emitting section 12a. Therefore, the overall illuminating range has a uniform brightness, thereby imparting balance of the brightness. As a result, it is possible to provide the capsule endoscope that enables to capture a good diagnosis image with the image capturing unit.

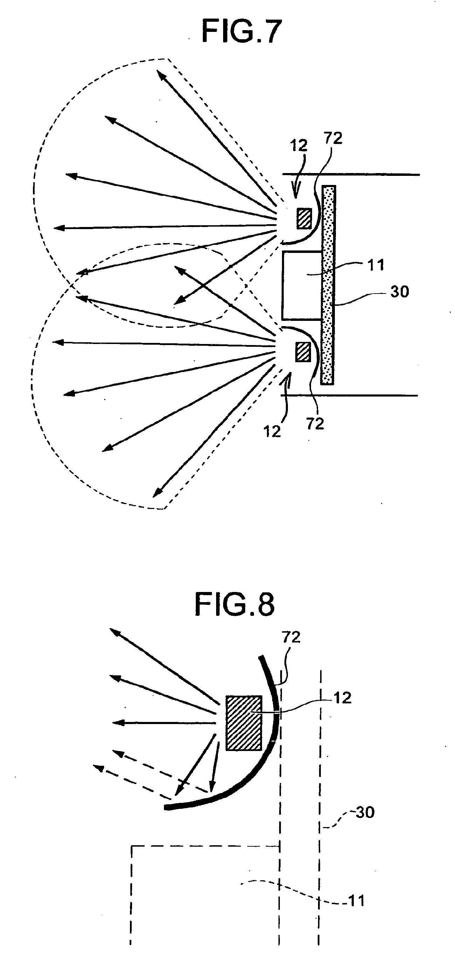

Fig. 7 is a schematic diagram of an illuminating unit of a capsule endoscope according to the invention. Fig. 8 is an enlarged view for explaining emission of a light from an illuminating unit of a capsule endoscope according to the invention.

As shown in Figs. 7 and 8, in this embodiment, instead of tilting the illuminating unit 12 with an inbuilt light emitting body 12a by using the tilting member 71 as in the second, a reflecting member 72 is provided on a back side of the illuminating unit 12. A part of emitting light that is emitted from the illuminating unit 12 is caused to reflect from the reflecting member 72, thereby changing orientation characteristics. Thus, a total of the light including outgoing light and reflected light is allowed to have an anisotropy factor of light.

According to this embodiment, not only the outgoing light but also a part of the outgoing light is caused to be reflected by using the reflecting member 72 on the back surface of the illuminating unit 12. Therefore, there is not change incorporated in the structure of the illuminating unit 12. The existing illuminating unit is used and the light is a total of the outgoing light and the reflected light. An observation central portion of the illuminating range is allowed to acquire a predetermined brightness by the light gathered from the plurality of light emitting sections and the illuminating range other than the observation central portion is allowed to acquire substantially the same or the same brightness as the predetermined brightness of the observation central portion by using a single light emitting body 12a. Therefore, the overall illuminating range has a uniform brightness, thereby imparting balance of the brightness. As a result, it is possible to provide the capsule endoscope that enables to capture a good diagnosis image with the image capturing unit.

Moreover, in this embodiment, the image capturing unit 11 is disposed in the observation central portion, and the illuminating units 12 are arranged around, and the number of illuminating units 12 is not limited in particular.

Furthermore, the illuminating unit 12 is not to be arranged around the image capturing unit 11 restrictedly and any structure in which the plurality of illuminating units 12 is used to illuminate uniformly an image capturing range in the capsule endoscope can be used for the illuminating unit.

Thus, according to the present invention, it is possible to provide a capsule endoscope that enables to capture a good diagnosis image by allowing a uniform illumination of overall illuminating plane by light.

Moreover, since an illuminating range becomes uniform by using this capsule endoscope, a good image can be captured, and it is possible to provide a capsule endoscope system that enables to contribute to an improved diagnostic analysis.

### INDUSTRIAL APPLICABILITY

The present invention is applicable to a capsule-shaped endoscope and a capsule endoscope system than can acquire good images of an inside of a body of a patient.

## Claims

1. A capsule endoscope (10) comprising:
an observation unit (11) having a predetermined observation range to observe an inside of a body of a patient (52) said observation range being centered on a central axis of said capsule endoscope;
a plurality of illuminating units (12) arranged around the observation unit (11), each of the illuminating units (12) emitting illuminating light (L) for illuminating the observation range of the observation unit (11); and
each of the illuminating units (12) has a light distribution characteristic that deviates from the center of the observation range of the observation unit (11),
the illuminating light emitted from the plurality of illuminating units (12) overlaps at a central portion of the observation range of the observation unit (11),
**characterised by** further comprising reflecting members (72) provided on backsides of each of the illuminating units (12) such that a part of the illuminating light from respective illuminating units (12) is directly emitted on the reflecting surfaces of the reflecting members (72) and a part of the illuminating light directly emitted on the reflecting surfaces is reflected from the reflecting surfaces in a direction away from the central axis of the capsule endoscope (10), wherein
each of the illuminating units (12) overlaping a part of the illuminating light reflected by the reflecting members (72) in addition to the illuminating light emitted from each of the illuminating units (12), at the central portion of the observation range, such that
a brightness at the central portion of the observation range illuminated by the plurality of light emitting units (12) and a brightness at portion that excludes the central portion of the observation range and that is illuminated by a single light emitting unit (12) are the same or substantially the same.

## Patentansprüche

1. Kapselendoskop (10) aufweisend:
eine Beobachtungseinheit (11) mit einem vorbestimmten Beobachtungsbereich zum Beobachten des Körperinneren eines Patienten (52), wobei der Beobachtungsbereich auf einer Mittelachse des Kapselendoskops zentriert ist;
eine Mehrzahl von Beleuchtungseinheiten (12), die um die Beobachtungseinheit (11) angeordnet sind, wobei jede der Beleuchtungseinheiten (12) Beobachtungslicht (L) zum Beleuchten des Beobachtungsbereichs der Beobachtungseinheit (11) emittiert; und
jede der Beleuchtungseinheiten (12) eine Lichtverteilungscharakteristik aufweist, die von dem Zentrum des Beobachtungsbereichs der Beobachtungseinheit (11) abweicht,
sich das von der Mehrzahl von Beleuchtungseinheiten (12) emittierte Beobachtungslicht bei einem zentralen Bereich des Beobachtungsbereichs der Beobachtungseinheit (11) überlappt,
**dadurch gekennzeichnet, dass** es ferner aufweist
reflektierende Elemente (72), die an Rückseiten jeder der Beleuchtungseinheiten (12) derart vorgesehen sind, dass ein Teil des Beleuchtungslichts von jeweiligen Beleuchtungseinheiten (12) direkt auf die reflektierenden Oberflächen der reflektierenden Elemente (72) emittiert wird und ein Teil des Beleuchtungslichts, welches direkt auf die reflektierenden Oberfläche emittiert wird, von den reflektierenden Oberflächen in eine Richtung weg von der Mittelachse des Kapselendoskops (10) reflektiert wird, wobei
jede der Beleuchtungseinheiten (12) bei dem zentralen Bereich des Beobachtungsbereichs einen Teil des von den reflektierenden Elementen (72) reflektierten Beleuchtungslichts zusätzlich zu dem von jedem der Beleuchtungseinheiten (12) emittierten Beleuchtungslicht überlappt, so dass
eine Helligkeit bei dem zentralen Bereich des Beobachtungsbereichs, der durch die Mehrzahl von Licht emittierenden Einheiten (12) beleuchtet wird, und eine Helligkeiten bei einem Bereich, der den zentralen Bereich des Beobachtungsbereichs ausschließt und der durch eine einzelne Licht emittierende Einheit (12) beleuchtet wird, gleich sind oder im Wesentlichen gleich sind.

## Revendications

1. Endoscope de type capsule (10) comprenant :
une unité d'observation (11) ayant une plage d'observation prédéterminée pour observer un intérieur d'un corps d'un patient (52) ladite plage d'observation étant centrée sur un axe central dudit endoscope de type capsule ;
une pluralité d'unités d'éclairage (12) agencées autour de l'unité d'observation (11), chacune des unités d'éclairage (12) émettant une lumière d'éclairage (L) destinée à éclairer la plage d'observation de l'unité d'observation (11) ; et
chacune des unités d'éclairage (12) possède une caractéristique de répartition de lumière qui dévie du centre de la plage d'observation de l'unité d'observation (11),
la lumière d'éclairage émise depuis la pluralité d'unités d'éclairage (12) se chevauche au niveau d'une partie centrale de la plage d'observation de l'unité d'observation (11),
**caractérisé en ce qu'**il comprend en outre des éléments réfléchissants (72) prévus à l'arrière de chacune des unités d'éclairage (12) d'une manière telle qu'une partie de la lumière d'éclairage provenant des unités d'éclairage respectives (12) est émise directement sur les surfaces réfléchissantes des éléments réfléchissants (72) et une partie de la lumière d'éclairage émise directement sur les surfaces réfléchissantes est réfléchie depuis les surfaces réfléchissantes dans une direction éloignée de l'axe central de l'endoscope de type capsule (10), dans lequel
chacune des unités d'éclairage (12) chevauchant une partie de la lumière d'éclairage réfléchie par les éléments réfléchissants (72) en plus de la lumière d'éclairage émise depuis chacune des unités d'éclairage (12), au niveau de la partie centrale de la plage d'observation, d'une manière telle qu'une luminosité au niveau de la partie centrale de la plage d'observation éclairée par la pluralité d'unités d'émission de lumière (12) et une luminosité au niveau d'une partie qui exclut la partie centrale de la plage d'observation et qui est éclairée par une unité d'émission de lumière unique (12) sont identiques ou sensiblement identiques.
